(19) Europäisches Patentamt
European Patent Office
Office européen des brevets

(11) Veröffentlichungsnummer : **0 029 993**
**B1**

# (12) EUROPÄISCHE PATENTSCHRIFT

(45) Veröffentlichungstag der Patentschrift : **13.10.82**

(51) Int. Cl.³ : **C 07 F 7/10**, A 01 N 55/00

(21) Anmeldenummer : **80107342.0**

(22) Anmeldetag : **25.11.80**

## (54) Silyl-benzimidazol-2-carbaminsäureester, Verfahren zu ihrer Herstellung und ihre Verwendung als Fungizide.

(30) Priorität : **04.12.79 DE 2948672**

(43) Veröffentlichungstag der Anmeldung : **10.06.81 (Patentblatt 81/23)**

(45) Bekanntmachung des Hinweises auf die Patenterteilung : **13.10.82 Patentblatt 82/41**

(84) Benannte Vertragsstaaten : **AT BE CH DE FR GB IT LI NL SE**

(56) Entgegenhaltungen :
**DE A 2 222 341**
**DE A 2 408 171**
**CHEMICAL ABSTRACTS, vol. 93, no. 1, 07-07-1980, Seite 760, Zusammenfassung 8238z Columbus, Ohio, USA JANZEN A.F. « N-(tert-Butyldimethylsilyl) imidazole and related heterocycles : carbon-13 nuclear magnetic resonance study and reaction with dimethylsulfoxide »**

(73) Patentinhaber : **BASF Aktiengesellschaft**
**Carl-Bosch-Strasse 38**
**D-6700 Ludwigshafen (DE)**

(72) Erfinder : **Acker, Rolf-Dieter, Dr.**
**Tuchbleiche 8**
**D-6906 Leimen (DE)**
Erfinder : **Koenig, Karl-Heinz, Dr.**
**Pierstrasse 8A**
**D-6710 Frankenthal (DE)**
Erfinder : **Hamprecht, Gerhard, Dr.**
**Rote-Turm-Strasse 28**
**D-6940 Weinheim (DE)**
Erfinder : **Pommer, Ernst-Heinrich, Dr.**
**Berliner Platz 7**
**D-6703 Limburgerhof (DE)**

**EP 0 029 993 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Silyl-benzimidazol-2-carbaminsäureester, Verfahren zu ihrer Herstellung und ihre Verwendung als Fungizide

Die Erfindung betrifft neue Silyl-benzimidazol-2-carbaminsäureester, Verfahren zu ihrer Herstellung, Fungizide, die diese Verbindungen als Wirkstoffe enthalten, sowie Verfahren zur Bekämpfung von Pilzen mit diesen Verbindungen.

Es ist bekannt, daß Benzimidazol-carbaminsäureester als Fungizide in Landwirtschaft und Gartenbau dienen (R. Wegler, Chemie der Pflanzenschutz- und Schädlingsbekämpfungsmittel, Bd. 4, Seite 180 ff., Springer-Verlag, Berlin, 1977). Die bekannten Verbindungen zeichnen sich durch gute systemische Wirksamkeit gegenüber einer Reihe von Pilzkrankheiten aus. Ihre Schwäche liegt jedoch in der Schwerlöslichkeit in vielen Lösungsmitteln, wodurch ihre Anwendung, z.B. im Holzschutz, begrenzt wird.

Es wurde gefunden, daß die neuen Silyl-benzimidazol-2-carbaminsäureester der allgemeinen Formel I

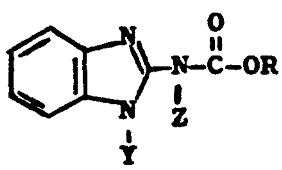

(I)

in der Y und Z für Wasserstoff oder einen Silylrest der Formel

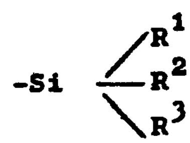

stehen,

wobei $R^1$, $R^2$ und $R^3$ unabhängig voneinander einen gegebenenfalls halogensubstituierten Alkyl-, Alkenyl- oder Alkinylrest mit bis zu 12 Kohlenstoffatomen, einen gegebenenfalls durch Alkyl oder Alkinyl mit bis zu 4 Kohlenstoffatomen substituierten Cycloalkylrest mit bis zu 7 Kohlenstoffatomen oder einen gegebenenfalls einfach oder mehrfach durch Halogen oder Alkyl mit bis zu 4 Kohlenstoffatomen substituierten Phenylrest bedeuten, und R für einen Alkylrest mit bis zu 4 Kohlenstoffatomen steht, mit der Maßgabe, daß Y und Z nicht gleichzeitig Wasserstoff bedeuten, eine wesentlich bessere fungizide Wirkung als bekannte Benzimidazol-carbaminsäureester aufweisen. Die neuen Verbindungen eignen sich insbesondere zur Bekämpfung von Schadpilzen aus den Klassen der Phycomyceten und Ascomyceten sowie aus der Sammelgruppe der Fungi imperfecti.

In der allgemeinen Formel I bedeutet R unverzweigtes oder verzweigtes Alkyl mit 1 bis 4 Kohlenstoffatomen, wie Methyl, Ethyl, n-Propyl, Isopropyl, n-Butyl, Isobutyl, tert.-Butyl.

$R^1$, $R^2$ und $R^3$ in den Silylresten, die für Y und Z in der allgemeinen Formel I stehen, bedeuten unverzweigtes oder verzweigtes, gegebenenfalls durch Halogen substituiertes Alkyl mit 1 bis 12 Kohlenstoffatomen, vorzugsweise 1 bis 4 Kohlenstoffatomen, unverzweigtes oder verzweigtes, gegebenenfalls durch Halogen substituiertes Alkenyl oder Alkinyl mit bis zu 12 Kohlenstoffatomen, vorzugsweise mit 3 oder 4 Kohlenstoffatomen, gegebenenfalls durch Alkyl- oder Alkinylgruppen mit bis zu 4 Kohlenstoffatomen substituiertes Cycloalkyl mit 3 bis 7 Kohlenstoffatomen oder gegebenenfalls durch Halogen oder Alkyl mit bis zu 4 Kohlenstoffatomen substituiertes Phenyl. Beispiele für diese Substituenten $R^1$, $R^2$ und $R^3$ sind Methyl, Ethyl, n-Propyl, Isopropyl, n-Butyl, Isobutyl, tert.-Butyl, n-Pentyl, l-Methyl-n-butyl, 1-Ethyl-n-propyl, Neopentyl, tert.-Amyl, 2-Methyl-n-butyl, 3-Methyl-n-butyl, Isoamyl, n-Hexyl, 1,3-Dimethyl-n-butyl, 2,3-Dimethyl-n-butyl, 1,1-Dimethyl-n-butyl, 1-Methyl-n-pentyl, 1-Ethyl-n-butyl, 1,2-Dimethyl-n-butyl, 3-Methyl-n-pentyl, 1-Methyl-1-ethyl-n-propyl, 4,4-Dimethyl-n-butyl, n-Heptyl, 1-Methyl-n-hexyl, 1-Ethyl-n-hexyl, 1-n-Propyl-n-butyl, n-Octyl, 1-Methyl-n-heptyl, 1-Ethyl-n-heptyl, 1-n-propyl-n-pentyl, 1-Methyl-4-ethyl-n-hexyl, 3-Methyl-1-isobutyl-n-butyl, 1-Isobutyl-4-ethyl-n-hexyl, 1-Ethyl-neopentyl, 1,2-Dimethyl-n-hexyl, 1-Methyl-4-ethyl-n-octyl, n-Nonyl, n-Decyl, n-Undecyl, n-Dodecyl, 1,4-Diethyl-n-octyl, 1,4-Diethyl-n-hexyl, 1,1-Dimethyl-isobutyl, 1,1-Dimethyl-n-pentyl, 1,1-Diethyl-n-propyl, 1-Methyl-1-ethyl-n-butyl, 1-Methyl-1-ethyl-isobutyl, 1,1-Dimethyl-isoamyl, 1,1-Dimethyl-neopentyl, 1,1-Dimethyl-n-hexyl, 1-n-Propyl-1-methyl-n-butyl, 1,1,3-Trimethyl-n-pentyl, 1,1-Dimethyl-n-heptyl, Cyclopropyl, Cyclobutyl, Cyclopentyl, Cyclohexyl, Cycloheptyl, 1-Methyl-cyclopentyl, 1-Methyl-cyclohexyl, 1-Ethyl-cycloexyl, 1-Ethinyl-cyclohexyl, Vinyl, Allyl, Methallyl, Crotyl, 2-Ethyl-n-hexen-2-yl, n-Hexen-5-yl, n-Undecen-10-yl, 2-Methyl-buten-2-yl,1-Methyl-isobuten-2-yl, 1-Methyl-n-propin-2-yl, n-Butin-2-yl, 1-Methyl-n-propen-2-yl, n-Propin-2-yl, 3-Methyl-n-buten-3-yl, 3-Methyl-n-buten-2-yl, 1,1-Dimethyl-n-propen-2-yl, Phenyl, o-, m-, p-Tolyl, o-, m-, p-Ethylphenyl, o-, m-, p-Propylphenyl, o-, m-, p-Isopropylphenyl, o-, m-, p-Butylphenyl, o-, m-, p-Isobutylphenyl, o-, m-, p-tert.-Butylphenyl, o-, m-, p-Chlorphenyl.

Man erhält die neuen Silyl-benzimidazol-2-carbaminsäureester der allgemeinen Formel I durch Umsetzung von Benzimidazol-carbaminsäureestern der allgemeinen Formel II

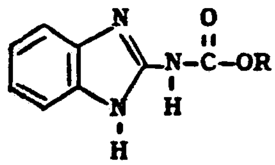
(II)

in der R die obengenannten Bedeutungen hat, oder Alkalimetall- oder Erdalkalimetallsalzen dieser Ester mit Halogensilanen der allgemeinen Formel III

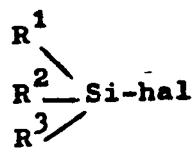
(III)

in der $R^1$, $R^2$ und $R^3$ die obengenannten Bedeutungen haben und Hal für Halogen steht, in Gegenwart eines inerten Verdünnungsmittels und gegebenenfalls in Gegenwart eines säurebindenden Mittels.

Verwendet man das Natriumsalz des Benzimidazol-2-carbaminsäure-methylesters und Triethylchlorsilan als Ausgangsmaterialien, so kann der Reaktionsablauf des erfindungsgemäßen Verfahrens durch das folgende Schema beschrieben werden :

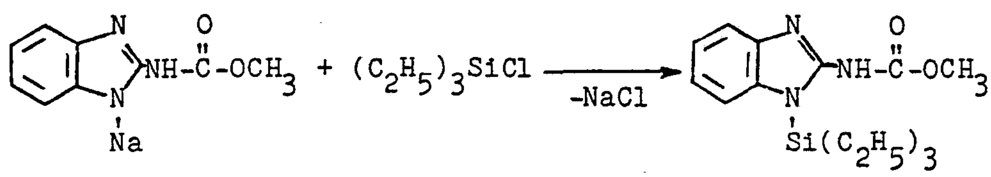


Verwendet man das Bis-Lithiumsalz des Benzimidazol-2-carbaminsäure-methylesters und n-Butyldimethylchlorsilan als Ausgangsmaterialien, so kann der Reaktionsablauf des erfindungsgemäßen Verfahrens durch folgendes Schema wiedergegeben werden :

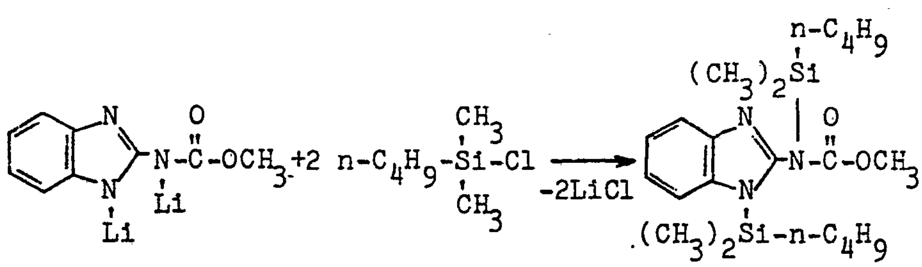


Bei dem Verfahren können beispielsweise folgende inerte Verdünnungsmittel verwendet werden : Formamide, wie Dimethylformamid, Dimethylacetamid ; Nitrile, wie Acetonitril, Benzonitril, Butyronitril ; Sulfoxide, wie Dimethylsulfoxid ; Phosphorsäureamide, wie Hexamethylphosphorsäuretriamid ; Ketone, wie Aceton, Ethylmethylketon, Cyclohexanon, Acetophenon ; Ether, wie Tetrahydrofuran, Anisol, Dimethyloxiethan, n-Butylethylether, Dioxan ; Nitroalkane, wie Nitromethan ; Nitrobenzol ; Harnstoffe, wie Tetramethylharnstoff ; Sulfone, wie Sulfolan ; Ester, wie Essigsäuremethylester, Propionsäuremethylester, Ameisensäuremethylester ; Halogenkohlenwasserstoffe, insbesondere Chlorokohlenwasserstoffe, beispielsweise Methylenchlorid, Chloroform, 1,2-Dichlorethan, 1,1,2,2- oder 1,1,1,2-Tetrachlorethan, Dichlorpropan, Trichlorethylen, Chlorbenzol, o-, m-, p-Dichlorbenzol, Fluorbenzol, o-, m-, p-Chlortoluol, Dichlornaphthalin, Tetrachlorkohlenstoff ; aliphatische oder cycloaliphatische Kohlenwasserstoffe, wie Heptan, Pinan, Benzinfraktionen innerhalb eines Siedepunktintervalles von 70 bis 190 °C, Cyclohexan, Methylcyclohexan, Dekalin, Petrolether, Ligroin, 2,2,4-Trimethylpentan, Octan ; aromatische und Kohlenwasserstoffe, wie Benzol, Toluol, o-, m-, p-Cymol, o-, m-, p-Xylol, Tetralin ; und entsprechende Gemische. Zweckmäßigerweise verwendet man das Lösungsmittel in einer Menge von 100 bis 2 000 Gew.%, vorzugsweise 100 bis 1 000 Gew.%, bezogen auf die Ausgangsverbindung der allgemeinen Formel II.

Als säurebindende Mittel kommen beispielsweise Alkalimetalle, Alkalihydride, Alkali- und Erdalkalialkoholate, metallorganische Verdindungen und tertiäre organische Amine in Betracht. Besonders geeignet sind Lithium, Lithiumhydrid, Natriumhydrid, Kaliumhydrid, Calciumhydrid, Penyllithium, n-Butyllithium, Methyllithium, Natriummethylat, Magnesiumethylat, Methylmagnesiumbromid, Ethylmagnesiumbromid, Phenylmagnesiumbromid, Phenylmagnesiumchlorid, Kaliummethylat, Natriumpropylat, Aluminiumisopropylat, Natriumbutylat, Lithiummethylat, Calciumcyclohexanolat, Natriumpropylat, Kalium-tert.-butylat, Trimethylamin, Triethylamin, Tripropylamin, Triisopropylamin, Tributylamin, Triisobutylamin, Tri-sec.-butylamin, Tri-tert.-butylamin, Tribenzylamin, Tricyclohexylamin, Triamylamin, Trihexylamin, N,N-Dimethylanilin, N,N-Diethylanilin, N,N-Diisopropylanilin, N,N-Dimethyltoluidin, N,N-Diethyltoluidin, N,N-Dipropyltoluidin, 4-N,N-Dimethylamino-pyridin, 4-N,N-Diethylamino-pyridin, N-Methylpiperidin, N-Ethylpiperidin, N-Methylpyrrolidin, N-Ethylpyrrolidin, N-Methylpyrrol, N-Methylmorpholin, N-Ethylmorpholin, N-Methylhexamethylenimin, Pyridin, Chinolin, α-, β-, γ-Picolin, Acridin, N,N,N',N'-Tetramethylethylendiamin, N-Ethyldiisopropylamin, N,N-Dimethylcyclohexylamin. Es können aber auch andere, üblicherweise verwendete basische Stoffe eingesetzt werden.

Es kann zweckmäßig sein, die Umsetzung in Gegenwart eines für Silylierungsreaktionen üblichen Reaktionsbeschleunigers durchzuführen. Geeignet sind beispielsweise Imidazol oder 4-Dimethylaminopyridin.

Die Umsetzung wird im allgemeinen bei −70 bis 100 °C, vorzugsweise bei −20 bis + 100 °C, während 30 Minuten und 200 Stunden, vorzugsweise 1 Stunde und 100 Stunden, drucklos oder unter Druck, diskontinuierlich oder kontinuierlich durchgeführt.

Im allgemeinen setzt man zur Herstellung der Monosilylverbindungen der allgemeinen Formel I pro Mol Verbindung der allgemeinen Formel II 0,5 bis 2 Mole, vorzugsweise 0,9 bis 1,5 Mole, der Verbindung der allgemeinen Formel III und gegebenenfalls 0,5 bis 2 Mol des säurebindenden Mittels ein. Verwendet man als säurebindendes Mittel ein Alkalimetall, ein Alkalihydrid, ein Alkali- oder Erdalkalialkoholat, so kann die Verbindung der allgemeinen Formel II zunächst in ihr Alkali- oder Erdalkalisalz überführt und in d..ser Form eingesetzt werden. Zur Herstellung der Bis-silylverbindungen der allgemeinen Formel I verwendet man auf 1 Mol Verbindung der allgemeinen Formel II 1 bis 4 Mole, vorzugsweise 1,8 bis 3 Mole, der Verdindung der allgemeinen Formel III.

Zur Vermeidung von Silylchlorid-Verlusten durch Hydrolyse empfiehlt es sich, die Umsetzung in Gegenwart eines Schutzgases, z.B. in Gegenwart von Stickstoff oder Argon, durchzuführen.

In einer bevorzugten Verfahrensweise wird das Chlorsilan der allgemeinen Formel III portionsweise einer Suspension der Verbindung der allgemeinen Formel II in einem geeigneten Lösungsmittel zugefügt ; es ist aber auch die portionsweise Zugabe der Suspension des Esters zum Chlorsilan möglich.

Die Endprodukte der allgemeinen Formel I können durch Einengen des Lösungsmittels, nach dem Abfiltrieren von Ungelöstem, isoliert werden ; im Rückstand kristallisiert dann die Verbindung der allgemeinen Formel I aus. Gegebenenfalls kann auch zur weiteren Reinigung in Lösungsmitteln, wie z.B. Tetrahydrofuran, Chloroform oder Cyclohexan aufgenommen werden, wobei nach erneuter Filtration das Lösungsmittel unter vermindertem Druck entfernt wird. Auch eine Reinigung durch Chromatographie ist möglich.

Die Ester der allgemeinen Formel II sowie Verfahren zu ihrer Herstellung sind aus R. Wegler, Chemie der Pflanzenschutz- und Schädlingsbekämpfungsmittel, Bd. 4, S. 180-185, Springer-Verlag, Berlin, 1977, bekannt. Die Halogensilane der allgemeinen Formel III sind nach üblichen Methoden herstellbar (V. Bazant et al, Organosilicon Compounds, Bd. 2/1, Academic Press, New York, 1965).

Die folgenden Beispiele erläutern die Herstellung der Silyl-benzimidazol-2-carbaminsäureester der allgemeinen Formel I. Die Mengenangaben sind Gewichtsteile.

## Beispiel 1

Zu einer Suspension von 10 Teilen Benzimidazol-2-carbaminsäure-methylester in 130 Teilen absolutem Tetrahydrofuran werden unter Stickstoff bei −78 °C 3,5 Teile n-Butyllithium in Hexan portionsweise zugefügt. Man erwärmt auf Raumtemperatur, läßt 5 Stunden rühren und fügt dann portionsweise 8,2 Teile Triethylchlorsilan zu. Nach 12 bis 15 Stunden Rühren wird vom Ungelösten filtriert und das Lösungsmittel unter vermindertem Druck entfernt. Im Rückstand kristallisieren 4,5 Teile N-Triethylsilyl-benzimidazol-2-carbaminsäuremethylester aus ; Zersetzung > 120 °C (Wirkstoff Nr. 1). NMR (δ in ppm) : 0,4-1,5 (15 H, $Si(C_2H_5)_3$) ; 3,70 (3H, $OCH_3$) ; 6,7-7,5 (4H, Aromat).

## Beispiel 2

Eine Lösung, hergestellt aus 4 Teilen Natrium in 40 Teilen Methanol, wird portionsweise zu einer Suspension von 30 Teilen Benzimidazol-2-carbaminsäure-methylester in 200 Teilen Dioxan zugefügt. Die Mischung wird 2 Stunden unter Rückfluß gekocht, das Lösungsmittel unter vermindertem Druck abdestilliert und der Rückstand scharf getrocknet.

Zu einer Suspension von 5 Teilen des so erhaltenen Natriumsalzes des Benzimidazolcarbaminsäuremethylesters in 15 Teilen Dimethylformamid werden 3,9 Teile Triethylchlorsilan bei Raumtemperatur protionsweise zugegeben. Nach 12 Stunden Rühren unter Stickstoffatmosphäre wird das Lösungsmittel unter vermindertem Druck destilliert, der Rückstand mit Tetrahydrofuran versetzt und die Lösung filtriert. Nach dem Abdestillieren des Lösungsmittels werden 2,7 Teile des N-Triethylsilyl-benzimidazol-2-carbaminsäure-methylesters erhalten ; Zersetzung > 120 °C (Wirkstoff Nr. 1).

Beispiel 3

5,0 Teile des in Beispiel 2 beschriebenen Natriumsalzes und 4,0 Teile Triethylchlorsilan werden in 120 Teilen Tetrahydrofuran bei Raumtemperatur unter Stickstoff zusammengegeben. Nach 12 Stunden Rühren wird filtriert. Nach dem Abdestillieren des Lösungsmittels erhält man 3,2 Teile des N-Triethylsilyl-benzimidazol-2-carbaminsäure-methylesters ; Zersetzung > 120 °C (Wirkstoff Nr. 1).

Beispiel 4

5 Teile Benzimidazol-2-carbaminsäure-methylester in 50 Teilen Tetrahydrofuran werden portionsweise mit 4,1 Teilen Triethylchlorsilan und 3,0 Teilen Triethylamin versetzt. Nach 12 Stunden wird abgesaugt und das Lösungsmittel unter vermindertem Druck abdestilliert. Man erhält 1,6 Teile des N-Triethylsilyl-benzimidazol-2-carbaminsäure-methylesters ; Zersetzung > 120 °C (Wirkstoff Nr. 1).

Beispiel 5

Zu einer Suspension von 10 Teilen Benzimidazol-2-carbaminsäure-methylester in 100 Teilen absolutem Tetrahydrofuran werden unter Stickstoff bei −78 °C 3,5 Teile n-Butyllithium in Hexan portionsweise zugefügt. Man erwärmt auf Raumtemperatur, rührt 6 Stunden und fügt dann portionsweise 9,5 Teile n-Butyl-dimethylchlorsilan zu. Nach 12 Stunden Rühren wird vom Ungelösten filtriert und das Lösungsmittel unter vermindertem Druck entfernt. Im Rückstand verbleiben 9.0 Teile N-Dimethyl-n-butylsilyl-benzimidazol-2-carbaminsäure-methylester ; Zersetzung >110 °C (Wirkstoff Nr. 2). NMR (δ in ppm) : 0,5-1,8 (n-$C_4H_9(CH_3)_2Si$, 15 H) 3,60 ($OCH_3$, 3H) ; 6,7-7,6 (Aromat, 4H).

Beispiel 6

5 Teile Benzimidazol-2-carbaminsäure-methylester werden in 80 Teilen absolutem Tetrahydrofuran suspendiert. Hierzu werden bei −78 °C unter Stickstoff portionsweise 1,8 Teile n-Butyllithium in Hexan zugefügt. Man läßt 2 Stunden bei 0 °C rühren und fügt erneut bei −78 °C portionsweise 1,8 Teile n-Butyllithium in Hexan zu. Nach 2 Stunden Rühren bei 0 °C werden 9 Teile n-Butyl-dimethylchlorsilan portionsweise zugefügt. Nach 12 Stunden Rühren bei Raumtemperatur wird vom Ungelösten filtriert und das Lösungsmittel unter vermindertem Druck entfernt. Man erhält 8,7 Teile N,N'-Bis-(n-butyldimethylsilyl)-benzimidazol-2-carbaminsäure-methylester (Wirkstoff Nr. 3).

NMR (δ in ppm) : 0,5-1,6 (n-$C_4H_9(CH_3)_2Si$, 30 H) ; 3,32 ($OCH_3$, 3 H) ; 6,6-7,5 (Aromat, 4 H).

Analog lassen sich beispielsweise folgende Verbindungen der allgemeinen Formel I herstellen :

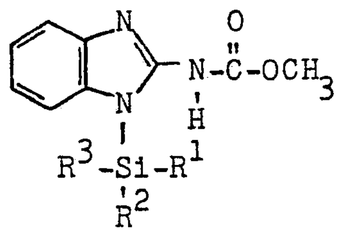

| Nr. | $R^1$ | $R^2$ | $R^3$ | NMR-Daten (δ in ppm) $SiR^1R^2R^3$ | $OCH_3$ | Aromat |
|---|---|---|---|---|---|---|
| 4 | $CH_3$ | $CH_3$ | t-$C_4H_9$ | 0,70; 1,00 | 3,80 | 6,7-7,5 |
| 5 | $CH_3$ | $CH_3$ | $C_2H_5$ | | | |
| 6 | $CH_3$ | $CH_3$ | n-$C_3H_7$ | | | |
| 7 | $CH_3$ | n-$C_3H_7$ | n-$C_3H_7$ | | | |
| 8 | $CH_3$ | $CH_3$ | i-$C_3H_7$ | | | |
| 9 | $CH_3$ | i-$C_3H_7$ | i-$C_3H_7$ | | | |
| 10 | $CH_3$ | n-$C_4H_9$ | n-$C_4H_9$ | | | |

| Nr. | $R^1$ | $R^2$ | $R^3$ | NMR-Daten (δ in ppm) $SiR^1R^2R^3$ | $OCH_3$ | Aromat |
|---|---|---|---|---|---|---|
| 11 | $n-C_4H_9$ | $n-C_4H_9$ | $n-C_4H_9$ | | | |
| 12 | $CH_3$ | $CH_3$ | $i-C_4H_9$ | | | |
| 13 | $CH_3$ | $i-C_4H_9$ | $i-C_4H_9$ | | | |
| 14 | $CH_3$ | $CH_3$ | $CH_3$ | | | |
| 15 | $CH_3$ | $t-C_4H_9$ | $t-C_4H_9$ | | | |
| 16 | $CH_3$ | $CH_3$ | Vinyl | | | |
| 17 | $CH_3$ | Vinyl | Vinyl | | | |
| 18 | $CH_3$ | $CH_3$ | Allyl | | | |
| 19 | $CH_3$ | Allyl | Allyl | | | |
| 20 | $CH_3$ | $CH_3$ | Ethinyl | | | |
| 21 | $CH_3$ | $CH_3$ | Propargyl | | | |
| 22 | $CH_3$ | $CH_3$ | Phenyl | 0,40; 7,0-7,5 | 3,70 | 6,5-7,8 |
| 23 | $CH_3$ | $C_2H_5$ | Phenyl | | | |
| 24 | $CH_3$ | Phenyl | Phenyl | | | |
| 25 | $CH_3$ | $CH_3$ | o-Tolyl | | | |
| 26 | $CH_3$ | $CH_3$ | m-Tolyl | | | |
| 27 | $CH_3$ | $CH_3$ | p-Tolyl | | | |
| 28 | $CH_3$ | $CH_3$ | p-Ethyl-phenyl | | | |
| 29 | $CH_3$ | $CH_3$ | m-Isopropyl-phenyl | | | |
| 30 | $CH_3$ | $CH_3$ | p-Isopropyl-phenyl | | | |
| 31 | $CH_3$ | $CH_3$ | m-Isobutyl-phenyl | | | |
| 32 | $CH_3$ | $CH_3$ | m-tert.-Butyl-phenyl | | | |
| 33 | $CH_3$ | $CH_3$ | p-tert.-Butyl-phenyl | | | |
| 34 | $CH_3$ | $CH_3$ | o-Chlorphenyl | | | |
| 35 | $CH_3$ | $CH_3$ | m-Chlorphenyl | | | |
| 36 | $CH_3$ | $CH_3$ | p-Chlorphenyl | | | |

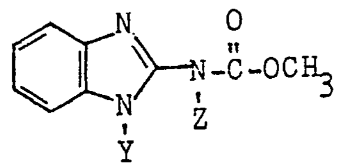

| Nr. | Y = $SiR^1R^2R^3$ $R^1$ | $R^2$ | $R^3$ | Z = $SiR^1R^2R^3$ $R^1$ | $R^2$ | $R^3$ | NMR-Daten (δ in ppm) $SiR^1R^2R^3$ | $OCH_3$ | Aromat |
|---|---|---|---|---|---|---|---|---|---|
| 37 | $C_2H_5$ | $C_2H_5$ | $C_2H_5$ | $C_2H_5$ | $C_2H_5$ | $C_2H_5$ | 0,4-1,5 | 3,32 | 6,6-7,4 |
| 38 | $CH_3$ | $CH_3$ | $C_2H_5$ | $CH_3$ | $CH_3$ | $C_2H_5$ | | | |
| 39 | $CH_3$ | $C_2H_5$ | $C_2H_5$ | $CH_3$ | $C_2H_5$ | $C_2H_5$ | | | |
| 40 | $CH_3$ | $CH_3$ | $CH_3$ | $CH_3$ | $CH_3$ | $CH_3$ | | | |
| 41 | $CH_3$ | $CH_3$ | n-$C_3H_7$ | $CH_3$ | $CH_3$ | n-$C_3H_7$ | | | |
| 42 | $CH_3$ | n-$C_3H_7$ | n-$C_3H_7$ | $CH_3$ | n-$C_3H_7$ | n-$C_3H_7$ | | | |
| 43 | $CH_3$ | i-$C_3H_7$ | i-$C_3H_7$ | $CH_3$ | i-$C_3H_7$ | i-$C_3H_7$ | | | |
| 44 | $CH_3$ | $CH_3$ | i-$C_3H_7$ | $CH_3$ | $CH_3$ | i-$C_3H_7$ | | | |
| 45 | i-$C_3H_7$ | i-$C_3H_7$ | i-$C_3H_7$ | i-$C_3H_7$ | i-$C_3H_7$ | i-$C_3H_7$ | | | |
| 46 | $CH_3$ | n-$C_4H_9$ | n-$C_4H_9$ | $CH_3$ | n-$C_4H_9$ | n-$C_4H_9$ | | | |
| 47 | n-$C_4H_9$ | n-$C_4H_9$ | n-$C_4H_9$ | n-$C_4H_9$ | n-$C_4H_9$ | n-$C_4H_9$ | | | |
| 48 | $CH_3$ | $CH_3$ | i-$C_4H_9$ | $CH_3$ | $CH_3$ | i-$C_4H_9$ | | | |
| 49 | $CH_3$ | i-$C_4H_9$ | i-$C_4H_9$ | $CH_3$ | i-$C_4H_9$ | i-$C_4H_9$ | | | |
| 50 | $CH_3$ | $CH_3$ | t-$C_4H_9$ | $CH_3$ | $CH_3$ | t-$C_4H_9$ | 0,55; 0,90 | 3,35 | 6,6-7,4 |
| 51 | $CH_3$ | t-$C_4H_9$ | t-$C_4H_9$ | $CH_3$ | t-$C_4H_9$ | t-$C_4H_9$ | | | |
| 52 | $CH_3$ | $CH_3$ | Vinyl | $CH_3$ | $CH_3$ | Vinyl | | | |
| 53 | $CH_3$ | Vinyl | Vinyl | $CH_3$ | Vinyl | Vinyl | | | |
| 54 | $CH_3$ | $CH_3$ | Allyl | $CH_3$ | $CH_3$ | Allyl | | | |
| 55 | $CH_3$ | Allyl | Allyl | $CH_3$ | Allyl | Allyl | | | |
| 56 | $CH_3$ | $CH_3$ | Ethinyl | $CH_2$ | $CH_2$ | Ethinyl | | | |
| 57 | $CH_3$ | $CH_3$ | Propargyl | $CH_3$ | $CH_3$ | Propargyl | | | |
| 58 | $CH_3$ | $CH_3$ | Phenyl | $CH_3$ | $CH_3$ | Phenyl | 0,40; 7,0-7,5 | 3,2 | 6,5-7,8 |
| 59 | $CH_3$ | $C_2H_5$ | Phenyl | $CH_3$ | $C_2H_5$ | Phenyl | | | |
| 60 | $CH_3$ | Phenyl | Phenyl | $CH_3$ | Phenyl | Phenyl | | | |
| 61 | $CH_3$ | $CH_3$ | o-Tolyl | $CH_3$ | $CH_3$ | c-Tolyl | | | |
| 62 | $CH_3$ | $CH_3$ | m-Tolyl | $CH_3$ | $CH_3$ | m-Tolyl | | | |
| 63 | $CH_3$ | $CH_3$ | p-Tolyl | $CH_3$ | $CH_3$ | p-Tolyl | | | |
| 64 | $CH_3$ | $CH_3$ | p-Ethyl-<br>-phenyl | $CH_3$ | $CH_3$ | p-Ethyl-<br>-phenyl | | | |
| 65 | $CH_3$ | $CH_3$ | m-Iso-<br>propylphenyl | $CH_3$ | $CH_3$ | m-Isopropyl-<br>phenyl | | | |
| 66 | $CH_3$ | $CH_3$ | p-Iso-<br>propylphenyl | $CH_3$ | $CH_3$ | p-Isopropyl-<br>phenyl | | | |

| Nr. | Y = $SiR^1R^2R^3$ $R^1$ | $R^2$ | $R^3$ | Z = $SiR^1R^2R^3$ $R^1$ | $R^2$ | $R^3$ | NMR-Daten (δ in ppm) $SiR^1R^2R^3$ | $OCH_3$ | Aromat |
|---|---|---|---|---|---|---|---|---|---|
| 67 | $CH_3$ | $CH_3$ | m-Iso-butylphenyl | $CH_3$ | $CH_3$ | m-Isobutyl-phenyl | | | |
| 68 | $CH_3$ | $CH_3$ | p-Iso-butylphenyl | $CH_3$ | $CH_3$ | p-Isobutyl-phenyl | | | |
| 69 | $CH_3$ | $CH_3$ | m-tert.-Butylphenyl | $CH_3$ | $CH_3$ | m-tert.-Butylphenyl | | | |
| 70 | $CH_3$ | $CH_3$ | p-tert.-Butylphenyl | $CH_3$ | $CH_3$ | p-tert.-Butylphenyl | | | |
| 71 | $CH_3$ | $CH_3$ | o-Chlor-phenyl | $CH_3$ | $CH_3$ | o-Chlor-phenyl | | | |
| 72 | $CH_3$ | $CH_3$ | m-Chlor-phenyl | $CH_3$ | $CH_3$ | m-Chlor-phenyl | | | |
| 73 | $CH_3$ | $CH_3$ | p-Chlor-phenyl | $CH_3$ | $CH_3$ | p-Chlor-phenyl | | | |

Die erfindungsgemäßen Wirkstoffe weisen eine starke fungitoxische Wirkung auf. Sie eignen sich insbesondere zur Bekämpfung von Schadpilzen aus den Klassen der Phycomyceten und Ascomyceten sowie aus der Sammelgruppe der Fungi imperfecti, so z.B. zur Bekämpfung von Plasmopara viticola an Reben, Phytophthora infestans an Tomaten und Kartoffeln, Erysiphe graminis an Weizen, Erysiphe cichoriacearum an Gurken, Uncinula necator an Reben, Podosphaera leucotricha an Äpfeln, Botrytis cinerea an Reben, Erdbeeren, Zierpflanzen und Solanaceen, Septoria nodorum an Weizen, Septoria glycines an Sojabohnen, Cercosporella herpotrichoides an Weizen, Cercospora personata an Erdnüssen, Diplodia natalensis an Citrus, Pullularia pullulans, Sclerophoma pityophila, Chaetomium globosum, Humicola grisea.

Beim Einsatz der neuen Verbindungen zum Schutz von Kulturpflanzen verwendet man zweckmäßigerweise aus ihnen hergestellte fungizide Mittel, die 0,1 bis 95 Gew.%, vorzugsweise 0,5 bis 90 Gew.%, Wirkstoff enthalten. Die Aufwandmengen liegen nach Art des gewünschten Effektes zwischen 0,01 und 3 kg oder mehr, vorzugsweise jedoch zwischen 0,01 und 1 kg Wirkstoff/ha.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Bei der Anwendung der Wirkstoffe im Materialschutz, z.B. als Fungizide für Anstrichfarben, betragen die Aufwandmengen 0,25-5 % Wirkstoff, bezogen auf das Gesamtgewicht der Farbe. Als weitere Materialien, die konserviert bzw. mikrozid ausgerüstet werden können, kommen Leime, Klebstoffe, Kunstsoffdispersionen, Dichtungsmasse, Papier, Textilien, Leder, Rohhäute, Kunststoffe, insbesondere Polyurethan und Weich-PVC in Betracht.

Die neuen Wirkstoffe können auch als fungizid wirksame Bestandteile öliger Holzschutzmittel zum Schutz von Holz gegen holzverfärbende Pilze eingesetzt werden ; die hier verwendeten Formulierungen enthalten in der Regel 0,5-2 Gew.% Wirkstoff. Die Anwendung erfolgt in der Weise, daß man das Holz mit diesen Mitteln behandelt, beispielsweise tränkt oder anstreicht.

Die erfindungsgemäßen Substanzen können in die üblichen Formulierungen, wie Lösungen, Emulsionen, Suspensionen, Stäube, Pulver, Pasten und Granulate, übergeführt werden. Die Anwendungsformen richten sich ganz nach den Verwendungszwecken ; sie sollten in jedem Fall eine feine und gleichmäßige Verteilung der wirksamen Substanzen gewährleisten. Die Formulierungen werden in bekannter Weise hergestellt, z.B. durch Verstrecken des Wirkstoffs mit Lösungsmitteln und/oder Trägerstoffen, ggf. unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen Lösungsmittel wie Aromaten, z.B. Xylol, Benzol, chlorierte Aromaten, z.B. Chlorbenzole, Paraffine, z.B. Erdölfraktionen, Alkohole, z.B. Methanol, Butanol, Amine, z.B. Ethanolamin, Dimethylformamid und Wasser ; Trägerstoffe,

wie natürliche Gesteinsmehle, z.B. Kaoline, Tonerden, Talkum, Kreide, und synthetische Gesteinsmehle, z.B. hochdisperse Kieselsäure, Silikate ; Emulgiermittel, wie nichtionogene und anionische Emulgatoren, z.B. Polyoxyethylen-Fettalkohol-Ether, Alkylsulfonate und Arylsulfonate, und Dispergiermittel, wie Lignin, Sulfitablaugen und Methylcellulose in Betracht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für Zubereitungen sind :

I. Man vermischt 90 Gew.-Teile des Wirkstoffs Nr. 1 mit 10 Gew.-Teilen N-Methyl-α-pyrrolidon und erhält eine Lösung, die zur Anwendung in Form kleinster Tropfen geeignet ist.

II. 10 Gew.-Teile der Verbindung des Wirkstoffs Nr. 1 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamin, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

III. 20 Gew.-Teile des Wirkstoffs Nr. 2 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

IV. 20 Gew.-Teile des Wirkstoffs Nr. 2 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

V. 80 Gew.-Teile des Wirkstoffs Nr. 3 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kielselsäuregel gut vermischt und in einer Hammermühle vermahlen. Durch feines Verteilen der Mischung in 20 000 Gew.-Teilen Wasser erhält man eine Spritzbrühe, die 0,4 Gew.-% des Wirkstoffs enthält.

VI. 3 Gew.-Teile des Wirkstoffs Nr. 4 werden mit 97 Gew.-Teilen feinteiligem Kaolin innig vermischt. Man erhält auf diese Weise ein Stäubemittel, das 3 Gew.-% des Wirkstoffs enthält.

VII. 30 Gew.-Teile des Wirkstoffs Nr. 37 werden mit einer Mischung aus 92 Gew.-Teilen pulverförmigem Kielselsäuregel und 8 Gew.-Teilen Paraffinöl, das auf die Oberfläche dieses Kieselsäuregels gesprüht wurde, innig vermischt. Man erhält auf diese Weise eine Aufarbeitung des Wirkstoffs mit guter Haftfähigkeit.

VIII. 40 Gew.-Teile des Wirkstoffs Nr. 50 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gew.-Teilen Wasser erhält man eine wäßrige Dispersion, die 0,04 Gew.-% Wirkstoff enthält.

IX. 20 Teile des Wirkstoffs Nr. 2 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die Wirkstoffe können auch mit anderen bekannten Fungiziden gemischt werden. In vielen Fällen erreicht man dabei auch eine Vergrößerung des fungiziden Wirkungsspektrums ; bei einer Anzahl dieser Fungizidimischungen treten auch synergistische Effekte auf, d.h. die fungizide Wirksamkeit des Kombinationsproduktes ist größer als die der addierten Wirksamkeiten der Einzelkomponenten.

Fungizide, die mit den erfindungsgemäßen Verbindungen kombiniert werden können, sind beispielsweise :

Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat,
Zinkethylenbisdithiocarbamat,
Tetramethylthiuramdisulfide,
Ammoniak-Komplex von Zink-(N,N-ethylen-bis-dithiocarbamat)
und
N,N'-Polyäthylen-bis-(thiocarbamoyl)-disulfid,
Zink-(N,N'-propylen-bis-dithiocarbamat),
Ammoniak-Komplex von Zink-(N,N'-propylen-bis-dithiocarbamat)
und
N,N'-Polypropylen-bis-(thiocarbamoyl)-disulfid ;

Nitroderivate, wie

Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;

heterocyclische Strukturen, wie

N-Trichlormethylthio-tetrahydrophthalamid,
N-Trichlormethylthio-phthalimid,
2-Heptadecyl-2-imidazolin-acetat,
2,4-Dichlor-6-(o-chloranilino)-s-triazin,
O,O-Diäthyl-phthalimidophosphonothionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-3-pyridinmethanol,
1,2-Bis-(3-ethoxycarbonyl-2-thioureido)-benzol,
1,2-Bis-(3-methoxycarbonyl-2-thioureido)-benzol

und verchiegene Fungizide, wie

Dodecylguanidinacetat,
3-(3-(3,5-Dimethyl-2-oxycyclohexyl)-2-hydroxyäthyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze.

5-Nitro-isophthalsäure-di-isopropylester,
1-(1',2',4'-Triazolyl-1')-[1-(4'-chlorphenoxy)]-3,3-dimethylbutan-2-on,
1-(1',2',4'-Triazolyl-1')-[1-(4'-chlorphenoxy)]-3,3-dimethylbutan-2-ol,
N-(n-Propyl)-N(2,4,6-trichlorphenoxyethyl)-N'-imidazolyl-harnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin.

Die folgenden Beispiele belegen die biologische Wirkung der neuen Verbindungen der allgemeinen Formel I. Vergleichsmittel ist der fungizide Wirkstoff 2-(Methoxy-carbonylamino)-benzimidazol (R. Wegler, Chemie der Pflanzenschutz- und Schädlingsbekämpfungsmittel, Bd. 4, S. 175, Springer-Verlag, Berlin, 1977).

Beispiel A

Wirksamkeit gegen Phytophthora infestans an Tomaten

Blätter von Topfpflanzen der Sorte « Große Fleischtomate » werden mit wäßrigen Suspensionen, die

0,05 und 0,25 % (Gewichtsprozent) Wirkstoff enthalten, besprüht. Nach dem Antrocknen des Spritzbelags werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18 °C aufgestellt. Nach 5 Tagen hat sich die Krankheit auf den unbehar. elten, jedoch infizierten Kontrollpflanzen so stark entwickelt, daß die fungizide Wirksamkeit der Substanzen beurteilt werden kann.

| Wirkstoff Nr. | Befall der Blätter nach Spritzung mit .. %iger Wirkstoffbrühe 0,05 | 0,025 |
|---|---|---|
| 2 | 2 | 2-3 |
| 4 | 1 | 2 |
| 50 | 1 | 1 |
| 2-(Methoxycarbonylamino)-<br>-benzimidazol<br>(Vergleichsmittel) | 4 | 5 |
| Kontrolle (unbehandelt) | 5 | |

0 = kein Pilzbefall, abgestuft bis 5 = Totalbefall

Beispiel B

Botrytis cinerea an Paprika

Paprikasämlinge der Sorte « Neusiel der Ideal Elite » werden, nachdem sich 4 bis 5 Blätter gut entwickelt haben, mit wäßrigen Suspensionen, die 0,1 bzw. 0,05 Gew.% einer Mischung aus 80 % Wirkstoff und 20 % Natriumligninsulfat enthalten, tropfnaß gespritzt. Nach dem Antrocken des Spritzbelags werden die Pflanzen mit einer Konidienaufschwemmung des Pilzes Botrytis cinerea besprüht und bei 22-24 °C in eine Kammer mit hoher Luftfeuchtigkeit gestellt, um optimale Bedingungen für die Pilzentwicklung zu erhalten. Nach 5 Tagen hat sich die Krankheit auf den unbehandelten Kontrollpflanzen so stark entwickelt, daß die entstandenen Blattnekrosen den überwiegenden Teil der Blätter bedecken.

| Wirkstoff Nr. | Blattnekrosen nach Spritzung mit .. %iger Spritzbrühe 0,1 | 0,05 |
|---|---|---|
| 2 | 0 | 1 |
| 3 | 0 | 1 |
| 4 | 0 | 1 |
| 50 | 0 | 1 |
| Kontrolle (unbehandelt) | 5 | |

0 = keine Nekrosen, abgestuft bis 5 = 2/3 der Blattfläche mit Nekrosen bedeckt

Beispiel C

Wirksamkeit gegen Chaetomium globosum und Pullularia pullulans

Filtrierpapierscheiben mit einem Durchmesser von 13 mm und einer Stärke von 1 mm werden mit 0,2 ml von Lösungen getränkt, die jeweils 800, 400 und 200 ppm Wirkstoff enthalten. Die Scheiben werden dann in Petrischalen auf einen 50 %igen Malzextraktagar, der zuvor getrennt mit Sporen (Konidien) der Pilze Chaetomium globosum und Pullularia pullulans beimpft wurde, aufgelegt. Anschließend werden die Schalen 3 Tage lang bei 22-24 °C bebrütet. Nach dieser Zeit haben sich die Pilze in den Kontrollschalen sehr gut entwickelt ; die fungizide Wirksamkeit der Wirkstoffe wird anhand der um die

Filtrierpapierscheiben herum entstandenen Hemmhöfe wie folgt beurteilt :

– kein Hemmhof (keine fungizide Wirksamkeit)
+ kleiner Hemmhof < 1 mm (geringe fungizide Wirksamkeit)
++ mittlerer Hemmhof 1-5 mm (gute fungizide Wirksamkeit)
+++ sehr großer Hemmhof > 5 mm (sehr gute fungizide Wirksamkeit)

| Wirkstoff Nr. | ... ppm Wirkstoff in der Tränklösung 800 | 400 | 200 |
|---|---|---|---|
| Chaetomium globosum | | | |
| 2 | +++ | +++ | +++ |
| 3 | +++ | +++ | +++ |
| 4 | +++ | +++ | +++ |
| 37 | +++ | +++ | +++ |
| 50 | +++ | +++ | +++ |
| Kontrolle (ohne Fungizid) | - | | |
| Pullularia pullulans | | | |
| 2 | +++ | +++ | +++ |
| 3 | +++ | +++ | +++ |
| 37 | +++ | +++ | +++ |
| 50 | +++ | +++ | +++ |
| Kontrolle (ohne Fungizid) | - | | |

**Ansprüche** (für die Vertragsstaaten : BE, CH, LI, DE, FR, GB, IT, NL, SE)

1. Silyl-benzimidazol-2-carbaminsäureester der allgemeinen Formel I

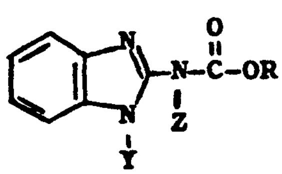

(I)

in der

Y und Z für Wasserstoff oder einen Silylrest der Formel

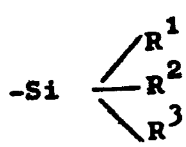

stehen, wobei

$R^1$, $R^2$ und $R^3$ unabhängig voneinander einen gegebenenfalls halogensubstituierten Alkyl-, Alkenyl- oder Alkinylrest mit bis zu 12 Kohlenstoffatomen, einen gegebenenfalls durch Alkyl oder Alkinyl mit bis zu 4 Kohlenstoffatomen substituierten Cycloalkylrest mit bis zu 7 Kohlenstoffatomen oder einen gegebenenfalls durch Halogen oder Alkyl mit bis zu 4 Kohlenstoffatomen substituierten Phenylrest bedeuten, und

R für einen Alkylrest mit bis zu 4 Kohlenstoffatomen steht, mit der Maßgabe, daß Y und Z nicht gleichzeitig Wasserstoff bedeuten.

2. Silyl-benzimidazol-2-carbaminsäureester der allgemeinen Formel I gemäß Anspruch 1, dadurch gekennzeichnet, daß Y und/oder Z für einen Silylrest der Formel

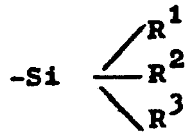

stehen, wobei

$R^1$, $R^2$ und $R^3$ unabhängig voneinander einen Alkylrest mit 1 bis 4 Kohlenstoffatomen bedeuten.

3. N-Triethylsilyl-benzimidazol-2-carbaminsäure-methylester.
4. N-(Dimethyl-n-butylsilyl)-benzimidazol-2-carbaminsäure-methylester.
5. N-(Dimethyl-t-butylsilyl)-benzimidazol-2-carbaminsäure-methylester.
6. N,N'-Bis-(triethylsilyl)-benzimidazol-2-carbaminsäure-methylester.
7. N,N'-Bis-(dimethyl-n-butylsilyl)-benzimidazol-2-carbaminsäure-methylester.
8. N,N'-Bis-(dimethyl-t-butylsilyl)-benzimidazol-2-carbaminsäure-methylester.
9. Verfahren zur Herstellung von Silyl-benzimidazol-2-carbaminsäureestern der allgemeinen Formel I gemäß Anspruch 1, dadurch gekennzeichnet, daß man Benzimidazol-carbaminsäureester der allgemeinen Formel II

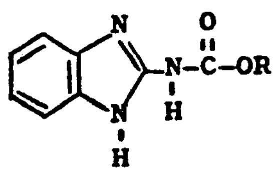

(II)

in der

R die im Anspruch 1 genannten Bedeutungen hat, oder Alkalimetall- oder Erdalkalimetallsalze dieser Ester in Gegenwart eines inerten Verdünnungsmittels mit Halogensilanen der allgemeinen Formel III

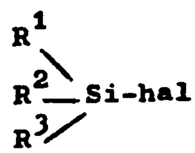

(III)

in der

$R^1$, $R^2$ und $R^3$ die im Anspruch 1 genannten Bedeutungen haben und Hal Halogen bedeutet, gegebenenfalls in Gegenwart eines säurebindenden Mittels bei einer Temperatur von − 70 bis + 100 °C umsetzt.

10. Fungizides Mittel, enthaltend einen Silyl-benzimidazol-2-carbaminsäureester der allgemeinen Formel I gemäß Anspruch 1.

11. Fungizides Mittel, enthaltend einen Silyl-benzimidazol-2-carbaminsäureester der allgemeinen Formel I gemäß Anspruch 1 und feste und/oder flüssige inerte Zusatzstoffe.

12. Verfahren zur Bekämpfung von Pilzen, dadurch gekennzeichnet, daß man eine fungizid wirksame Menge eines Silyl-benzimidazol-2-carbaminsäureesters der allgemeinen Formel I gemäß Anspruch 1 auf diese bzw. auf durch Pilzbefall bedrohte Flächen, Pflanzen oder Saatgüter einwirken läßt.

**Ansprüche** (für den Vertragsstaat AT)

1. Fungizides Mittel, enthaltend einen Silyl-benzimidazol-2-carbaminsäureester der allgemeinen Formel I

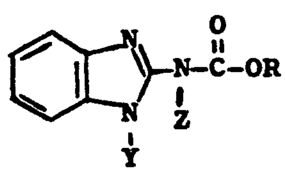

(I)

in der

Y und Z für Wasserstoff oder einen Silylrest der Formel

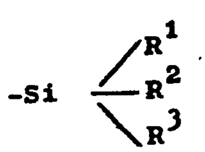

stehen, wobei

$R^1$, $R^2$ und $R^3$ unabhängig voneinander einen gegebenenfalls halogensubstituierten Alkyl-, Alkenyl- oder Alkinylrest mit bis zu 12 Kohlenstoffatomen, einen gegebenenfalls durch Alkyl oder Alkinyl mit bis zu 4 Kohlenstoffatomen substituierten Cycloalkylrest mit bis zu 7 Kohlenstoffatomen oder einen gegebenenfalls durch Halogen oder Alkyl mit bis zu 4 Kohlenstoffatomen substituierten Phenylrest bedeuten, und

R für einen Alkylrest mit bis zu 4 Kohlenstoffatomen steht, mit der Maßgabe, daß Y und Z nicht gleichzeitig Wasserstoff bedeuten, als Wirkstoff.

2. Fungizides Mittel, enthaltend einen Silyl-benzimidazol-2-carbaminsäureester der allgemeinen Formel I gemäß Anspruch 1, dadurch gekennzeichnet, daß Y und/oder Z für einen Silylrest der Formel

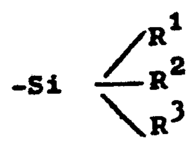

stehen, wobei

$R^1$, $R^2$ und $R^3$ unabhängig voneinander einen Alkylrest mit 1 bis 4 Kohlenstoffatomen bedeuten.

3. Fungizides Mittel, enthaltend den N-(Dimethyl-n-butylsilyl)-benzimidazol-2-carbaminsäure-methylester als Wirkstoff.

4. Fungizides Mittel, enthaltend den N-(Dimethyl-t-butylsilyl)-benzimidazol-2-carbaminsäure-methylester als Wirkstoff.

5. Fungizides Mittel, enthaltend den N-(Dimethyl-phenylsilyl)-benzimidazol-2-carbaminsäuremethylester als Wirkstoff.

6. Fungizides Mittel, enthaltend den N,N'-Bis-(triethylsilyl)-benzimidazol-2-carbaminsäure-methylester als Wirkstoff.

7. Fungizides Mittel, enthaltend den N,N'-Bis-(dimethyl-n-butylsilyl)-benzimidazol-2-carbaminsäure-methylester als Wirkstoff.

8. Fungizides Mittel, enthaltend den N,N'-Bis-(dimethyl-t-butylsilyl)-benzimidazol-2-carbaminsäure-methylester als Wirkstoff.

9. Verfahren zur Herstellung von Silyl-benzimidazol-2-carbaminsäureestern der allgemeinen Formel I gemäß Anspruch 1, dadurch gekennzeichnet, daß man Benzimidazol-carbaminsäureester der allgemeinen Formel II

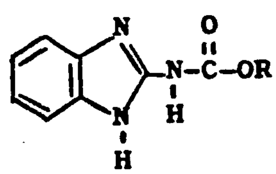

(II)

in der

R die im Anspruch 1 genannten Bedeutungen hat, oder Alkalimetall- oder Erdalkalimetallsalze dieser Ester in Gegenwart eines inerten Verdünnungsmittels mit Halogensilanen der allgemeinen Formel III

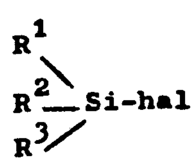

(III)

in der

$R^1$, $R^2$ und $R^3$ die im Anspruch 1 genannten Bedeutungen haben und Hal Halogen bedeutet, gegebenenfalls in Gegenwart eines säurebindenten Mittels bei einer Temperatur von – 70 bis + 100 °C umsetzt.

**Claims** (for the contracting States : BE, CH, LI, DE, FR, GB, IT, NL, SE)

1. A silyl-benzimidazole-2-carbamic acid ester of the general formula I

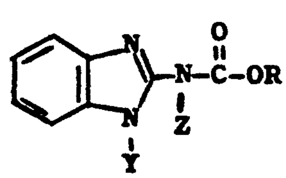

(I)

where

Y and Z are hydrogen or a silyl radical of the formula

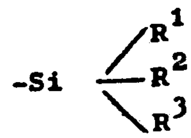

where

$R^1$, $R^2$ and $R^3$ independently of one another are unsubstituted or halogen-substituted alkyl, alkenyl or alkynyl of up to 12 carbon atoms, cycloalkyl of up to 7 carbon atoms which is unsubstituted or substituted by alkyl or alkynyl of up to 4 carbon atoms, or phenyl which is unsubstituted or substituted by halogen or alkyl of up to 4 carbon atoms, and R is alkyl of up to 4 carbon atoms, with the proviso that Y and Z are not both hydrogen.

2. A silyl-benzimidazole-2-carbamic acid ester of the general formula I as claimed in claim 1, characterized in that Y and/or Z denote a silyl radical of the formula

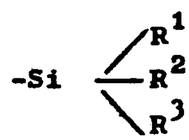

where

$R^1$, $R^2$ and $R^3$ independently of one another are alkyl of from 1 to 4 carbon atoms.

3. Methyl N-triethylsilyl-benzimidazole-2-carbamate.

4. Methyl N-(dimethyl-n-butylsilyl)-benzimidazole-2-carbamate.

5. Methyl N-(dimethyl-tert-butylsilyl)-benzimidazole-2-carbamate.

6. Methyl N,N'-bis-(triethylsilyl)-benzimidazole-2-carbamate.

7. Methyl N,N'-bis-(dimethyl-n-butylsilyl)-benzimidazole-2-carbamate.

8. Methyl N,N'-bis-(dimethyl-tert-butylsilyl)-benzimidazole-2-carbamate.

9. A process for the preparation of a silyl-benzimidazole-2-carbamic acid ester of the general formula I as claimed in claim 1, characterized in that a benzimidazole-carbamic acid ester of the general formula II

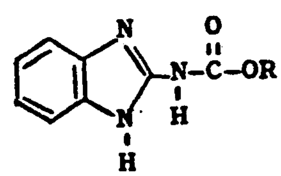

(II)

where

R has the meanings given in claim 1, or an alkali metal salt or alkaline earth metal of such an ester, is reacted with a halosilane of the general formula III

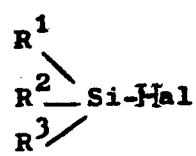

(III)

where

$R^1$, $R^2$ and $R^3$ have the meanings given in claim 1 and Hal is halogen, in the presence of an inert diluent and in the presence or absence of an acid acceptor at a temperature of from $-70$ to $+100$ °C.

10. A fungicidal agent containing a silyl-benzimidazole-2-carbamic acid ester of the general formula I as claimed in claim 1.

11. A fungicidal agent containing a silyl-benzimidazole-2-carbamic acid ester of the general formula I as claimed in claim 1 and solid and/or liquid inert additives.

12. A process for combating fungi, characterized in that a fungicidally effective amount of a silyl-benzimidazole-2-carbamic acid ester of the general formula I as claimed in claim 1 is allowed to act on the fungi, or areas, plants or seed threatened by fungus attack.

**Claims** (for the contracting State AT)

1. A fungicidal agent containing, as active ingredient, a silyl-benzimidazole-2-carbamic acid ester of the general formula I

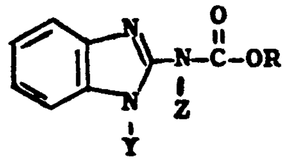 (I)

where
Y and Z are hydrogen or a silyl radical of the formula

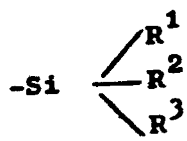

where
$R^1$, $R^2$ and $R^3$ independently of one another are unsubstituted or halogen-substituted alkyl, alkenyl or alkynyl of up to 12 carbon atoms, cycloalkyl of up to 7 carbon atoms which is unsubstituted or substituted by alkyl or alkynyl of up to 4 carbon atoms, or phenyl which is unsubstituted or substituted by halogen or alkyl of up to 4 carbon atoms, and R is alkyl of up to 4 carbon atoms, with the proviso that Y and Z are not both hydrogen.

2. A fungicidal agent containing a silyl-benzimidazole-2-carbamic acid ester of the general formula I as claimed in claim 1, characterized in that Y and/or Z denote a silyl radical of the formula

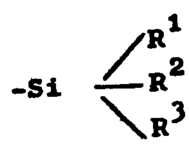

where
$R^1$, $R^2$ and $R^3$ independently of one another are alkyl of from 1 to 4 carbon atoms.

3. A fungicidal agent containing methyl N-(dimethyl-n-butylsilyl)-benzimidazole-2-carbamate as active ingredient.

4. A fungicidal agent containing methyl N-(dimethyl-tert-butylsilyl)-benzimidazole-2-carbamate as active ingredient.

5. A fungicidal agent containing N-(dimethyl-phenylsilyl)-benzimidazole-2-carbamate as active ingredient.

6. A fungicidal agent containing methyl N,N'-bis-(triethylsilyl)-benzimidazole-2-carbamate as active ingredient.

7. A fungicidal agent containing methyl N,N'-bis-(dimethyl-n-butylsilyl)-benzimidazole-2-carbamate as active ingredient.

8. A fungicidal agent containing methyl N,N'-bis-(dimethyl-tert-butylsilyl)-benzimidazole-2-carbamate as active ingredient.

9. A process for the preparation of a silyl-benzimidazole-2-carbamic acid ester of the general formula I as claimed in claim 1, characterized in that a benzimidazole-carbamic acid acid ester of the general formula II

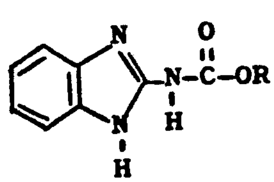 (II)

where
R has the meanings given in claim 1, or an alkali metal salt or alkaline earth metal salt of such an

ester, is reacted with a halosilane of the general formula III

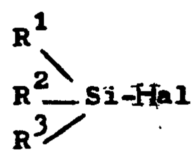 (III)

where

$R^1$, $R^2$ and $R^3$ have the meanings given in claim 1 and Hal is halogen, in the presence of an inert diluent and in the presence or absence of an acid acceptor at a temperature of from − 70 to + 100 °C.

**Revendications** (pour les Etats contractants : BE, CH, LI, DE, FR, GB, IT, NL, SE)

1. Ester d'acide silylbenzimidazol-2-carbamique de formule générale I

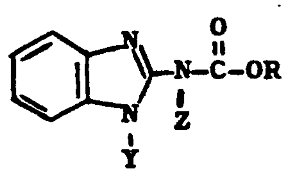 (I)

dans laquelle

Y et Z représentent hydrogène ou un reste silyle de formule

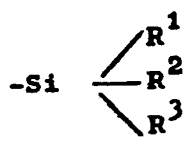

$R^1$, $R^2$ et $R^3$ représentent, indépendamment les uns des autres, un reste alkyle, alcényle ou alcynyle ayant jusqu'à 12 atomes de carbone, éventuellement substitué par halogène, un reste cycloalkyle ayant jusqu'à 7 atomes de carbone, éventuellement substitué par alkyle ou alcynyle ayant jusqu'à 4 atomes de carbone, ou un reste phényle éventuellement substitué par halogène ou alkyle ayant jusqu'à 4 atomes de carbone, sous réserve que Y et Z ne représentent pas simultanément hydrogène,

R désigne un reste alkyle ayant jusqu'à 4 atomes de carbone.

2. Ester d'acide silyl-benzimidazol-2-carbamique de formule générale I selon la revendication 1, caractérisé par le fait que Y et/ou Z représentent un reste silyle de formule

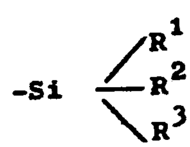

$R^1$, $R^2$ et $R^3$ représentant, indépendamment les uns des autres, un reste alkyle en $C_1$ à $C_4$.

3. N-triéthylsilyl-benzimidazol-2-carbamate de méthyle.

4. N-(diméthyl-n-butyl-silyl)-benzimidazol-2-carbamate de méthyle.

5. N-(diméthyl-t-butyl-silyl)-benzimidazol-2-carbamate de méthyle.

6. N,N'-bis-(triéthyl-silyl)-benzimidazol-2-carbamate de méthyle.

7. N,N'-bis-(diméthyl-n-butylsilyl)-benzimidazol-2-carbamate de méthyle.

8. N,N'-bis-(diméthyl-t-butylsilyl)-benzimidazol-2-carbamate de méthyle.

9. Procédé de préparation d'esters d'acide silyl-benzimidazol-2-carbamique de formule générale I selon la revendication 1, caractérisé par le fait que l'on fait réagir, à une température de − 70 à + 100 °C, en présence d'un diluant inerte et éventuellement en présence d'un liant d'acide, des esters d'acide benzimidazol-carbamique de formule générale II

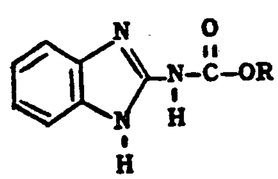 (II)

dans laquelle

R a les significations données dans la revendication 1, ou des sels de métaux alcalins ou alcalino-terreux de ces esters, avec des halogènes-silanes de formule générale III

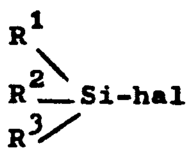

(III)

dans laquelle

$R^1$, $R^2$ et $R^3$ ont les significations données dans la revendication 1, hal représentant halogène.

10. Fongicide contenant un ester d'acide silyl-benzimidazol-2-carbamique de formule générale I selon la revendication 1.

11. Fongicide contenant un e.ter d'acide silyl-benzimidazol-2-carbamique de formule générale I selon la revendication 1 et des additifs inertes solides et/ou liquides.

12. Procédé de lutte contre des champignons, caractérisé par le fait qu'on fait agir une quantité active, du point de vue fongicide, d'un ester d'acide silyl-benzimidazole-2-carbamique de formule générale I selon la revendication 1 sur ces champignons ou sur les surfaces, plantes ou semences menacées par l'attaque de champignons.

**Revendications** (pour l'Etat contractant AT)

1. Fongicides contenant, comme principe actif, un ester d'acide silylbenzimidazol-2-carbamique de formule générale I

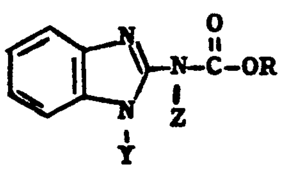

(I)

dans laquelle

Y et Z représentent hydrogène ou un reste silyle de formule

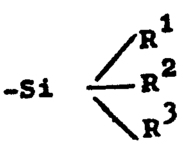

$R^1$, $R^2$ et $R^3$ représentant, indépendamment les uns des autres, un reste alkyle, alcényle ou alcynyle ayant jusqu'à 12 atomes de carbone, éventuellement substitué par halogène, un reste cycloalkyle ayant jusqu'à 7 atomes de carbone, éventuellement substitué par alkyle ou alcynyle ayant jusqu'à 4 atomes de carbone, ou un reste phényle éventuellement substitué par halogène ou alkyle ayant jusqu'à 4 atomes de carbone, sous réserve que Y et Z ne représentent pas simultanément hydrogène,

R désigne un reste alkyle ayant jusqu'à 4 atomes de carbone.

2. Fongicide contenant un ester d'acide silyl-benzimidazol-2-carbamique de formule générale I selon la revendication 1, caractérisé par le fait que Y et/ou Z représentent un reste silyle de formule

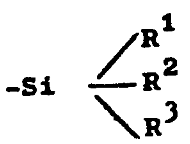

$R^1$, $R^2$ et $R^3$ représentant, indépendamment les uns des autres, un reste alkyle en $C_1$ à $C_4$.

3. Fongicide contenant, comme principe actif, le N-(diméthyl-n-butyl-silyl)-benzimidazol-2-carbamate de méthyle.

4. Fongicide contenant, comme principe actif, le N-(diméthyl-t-butyl-silyl)-benzimidazol-2-carbamate de méthyle.

5. Fongicide contenant, comme principe actif, le N-(diméthyl-phényl-silyl)-benzimidazol-2-carbamate de méthyle.

6. Fongicide contenant, comme principe actif, le N,N'-bis-(triéthyl-silyl)-benzimidazol-2-carbamate de méthyle.

7. Fongicide contenant, comme principe actif, le N,N'-bis-(diméthyl-n-butylsilyl)-benzimidazol-2-carbamate de méthyle.

8. Fongicide contenant, comme principe actif, le N,N'-bis-(diméthyl-t-butylsilyl)-benzimidazol-2-carbamate de méthyle.

9. Procédé de préparation d'esters d'acide silyl-benzimidazol-2-carbamique de formule générale I selon la revendication 1, caractérisé par le fait que l'on fait réagir, à une température de − 70 à + 100 °C, en présence d'un diluant inerte et éventuellement en présence d'un liant d'acide, des esters d'acide benzimidazol-carbamique de formule générale II

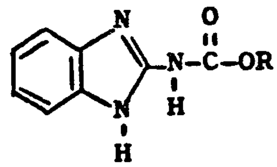

(II)

dans laquelle

R a les significations données dans la revendication 1, ou des sels de métaux alcalins ou alcalino-terreux de ces esters, avec des halogènes-silanes de formule générale III

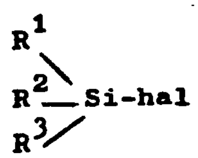

(III)

dans laquelle

$R^1$, $R^2$ et $R^3$ ont les significations données dans la revendication 1, hal représentant halogène.